# EUROPÄISCHE PATENTSCHRIFT

(11) **EP 0 780 444 B1**
(45) Veröffentlichungstag und Bekanntmachung des Hinweises auf die Patenterteilung: **05.09.2001**
(21) Anmeldenummer: 96119778.7
(22) Anmeldetag: 10.12.1996
(51) Int. Cl.: C09B 57/12, C07D 487/06, C07D 471/06, C08K 5/3442

(54) **Verfahren zur Herstellung polycyclischer Verbindungen**
Process to manufacture polycyclic compounds
Procédé de fabrication de composés polycycliques

(30) Priorität: 22.12.1995 DE 19548453
(43) Veröffentlichungstag der Anmeldung: 25.06.1997
(73) Patentinhaber: BAYER AG, 51368 Leverkusen (DE)
(72) Erfinder: Pelster, Thomas, Dr., 50999 Köln (DE); Kalz, Dietmar, Dr., 53819 Neunkirchen-Seelscheid (DE)

(56) Entgegenhaltungen:
- EP-A- 0 367 067
- EP-A- 0 570 800
- FR-A- 2 272 152
- DATABASE WPI Section Ch, Week 7735 Derwent Publications Ltd., London, GB; Class A60, AN 77-61871Y XP002073312 & JP 52 085 218 A (YAMAMOTO KAGAKU GOSEI KK)
- DATABASE WPI Section Ch, Week 8827 Derwent Publications Ltd., London, GB; Class E13, AN 88-186142 XP002073313 & JP 63 122 668 A (KAWASAKI KASEI KOGYO KK)
- LIFENTSEV ET AL: "dyeing by the synthesis of pigments on the material" TECHNOLOGY OF THE TEXTILE INDUSTRY, Nr. 1, 1968, Seiten 106-110, XP002073311 su

## Beschreibung

Die vorliegende Erfindung betrifft ein neues Verfahren zur Herstellung polycyclischer Verbindungen sowie die Verwendung der so erhaltenen polycyclischen Verbindungen zum Färben von synthetischen hydrophoben Materialien.

Es ist bereits bekannt, dass polycyclische Verbindungen wie Perinone in diversen Lösungsmitteln sowie in der Schmelze aus den entsprechenden aromatischen Dicarbonsäuren und Diaminen herstellbar sind. (DE-A-24 24 542, BE-A-600 302, DE-A-22 36 555, DE-A-21 57 547, DE-A-43 39 699 DE-A-43 27 555 und EP-A-570 800).

Gemäß der Derwent Publikation Class E13, AN 88-18614Z wird ein Verfahren zur Umsetzung von aromatischen Diaminen mit Phthalsäurederivaten in einem nicht wässrigen Medium offenbart.

Gemäß der Derwent Publikation Class A60, AN77-61871Y wird ein Verfahren zur Pigmentherstellung beschrieben, wobei Tetracarbonsäuren mit Phenylendiaminen umgesetzt werden.

Aus JP-A-53 21 221 ist weiterhin bekannt, die Herstellung derartiger Perinone in wässrigen Reaktionsmedien wie wässriger Salzsäure und in Gegenwart eines nicht-ionischen oder kationischen oberflächenaktiven Mittels ablaufenzulassen. In JP-A-52 85 218 erfolgt die Umsetzung zu Perinonen ebenfalls in einem wässrigen Reaktionsmedium, enthaltend Salzsäure und ein Benetzungsmittel (wetting agent). Als typische Benetzungsmittel werden Alkohole wie beispielsweise Methanol, Ethanol, Isopropanol, Ethylenglykol oder Glycerin genannt. Bei dem zuletzt genannten Verfahren wird zwar das organische durch ein wässriges Reaktionsmedium ersetzt, was jedoch nicht zu Produkten in der gewünschten Reinheit führt. So ist beispielsweise der Gehalt an nicht umgesetztem Diamin und kunststoffunlöslichen organischen Rückständen, wie z.B. Imiden, die als Nebenprodukte aus den eingesetzten Diaminen und Dicarbonsäurederivaten entstehen können, im erhaltenen Produkt zu hoch, so dass vor seiner weiteren Verwendung aufwendige, nachgeschaltete Reinigungsschritte erforderlich werden.

Es wurde ein Verfahren zur Herstellung von Verbindungen der Formel (I) 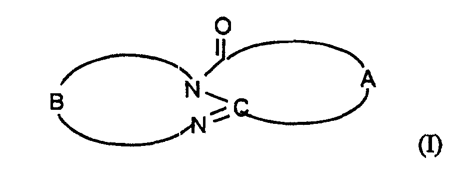 durch Umsetzung von aromatischen Dicarbonsäuren der Formel (II) 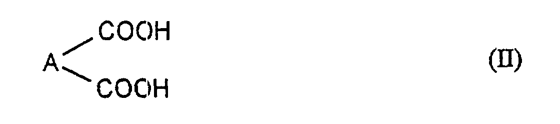 bzw. deren Anhydriden und/oder Ester mit aromatischen Diaminen der Formel (III) 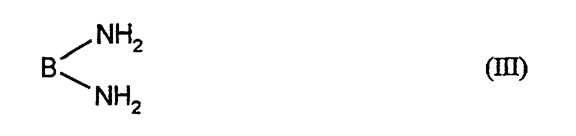 gefunden, worin
- A und B: unabhängig voneinander ortho-Phenylen, ortho-Naphthylen, peri-(1,8)-Naphthylen oder Arylen aus mehr als zwei miteinander kondensierten Benzolringen, das gegebenenfalls substituiert ist, bedeuten, wobei die Arylenreste A und B in Formel (I), die mehr als zwei miteinander kondensierter Benzolringe aufweisen, in ortho- oder entsprechend einer peri-Stellung im Naphthylen verbrückt sind,
das dadurch gekennzeichnet ist, daß die Umsetzung von (II) und (III) in einem Reaktionsmedium erfolgt, welches Wasser und eine hydrotrope Verbindung, die eine relative Dielektrizitätskonstante ε von 20 bis 60, vorzugsweise 30-50 (bei 25°C) besitzt, enthält.

Die relative Dielektrizitätskonstante ε eines Stoffes bei einer bestimmten Temperatur ist das Verhältnis der Kapazität eines mit dem Stoff gefüllten Kondensators zu der Kapazität des gleichen Kondensators im Vakuum bei dieser Temperatur (s. dazu auch Lexikon der Physik, Bd. 1, Hermann Franke, Franckhsche Verlagshandlung, Stuttgart 1969).

Die peri-Stellung entspricht eigentlich der 1,8-Stellung im Naphthalin. Die Bedeutung wird allerdings sowohl in der Literatur als auch im Sinne der vorliegenden Anmeldung auch auf Arylene ausgeweitet, die mehr als zwei miteinander kondensierte Benzolringe aufweisen.

Als mögliche Substituenten für A kommen beispielsweise ein oder mehrere gleiche oder verschiedene Substituenten Y in Frage, wobei Y = C₁-C₆-Alkyl, Halogen, Nitro, C₆-C₁₀-Aryl, C₆-C₁₀-Aryloxysulfonyl, Hydroxy, C₁-C₄-Alkoxy, C₆-C₁₀-Aryloxy, ein gegebenenfalls durch C₁-C₆-Alkyl oder C₆-C₁₀-Aryl substituiertes Aminosulfonyl oder einen ankondensierten cycloaliphatischen oder heterocyclischen Rest bedeutet.

Besonders bevorzugte Substituenten X sind Chlor, Fluor, Brom, Nitro, Methoxy, NH₂, Benzyloxy, Hydroxy, -SO₂O(C₆H₅), -SO₂N(CH₃)₂, -SO₂NHCH₃, Methyl, Ethyl, n-Propyl, iso-Propyl, n-, sec.-, tert.-Butyl, NHCOCH₃, -N(C₂H₅)₂ oder gegebenenfalls substituiertes Phenyl.

Als mögliche Substituenten für B kommen beispielsweise ein oder mehrere gleiche oder verschiedene Substituenten X in Frage, wobei X die Bedeutung von Y annehmen kann, davon jedoch unabhängig ist

In einer bevorzugten Ausführungsform des erfindungsgemäßen Verfahrens, werden Verbindungen der Formel (I) hergestellt, die der Formel (IV) entsprechen 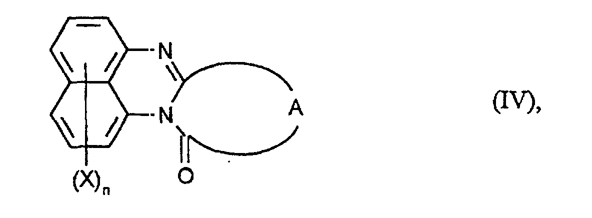 worin
- A und X: die obengenannten Bedeutungen haben und
- n: für eine Zahl von Null bis 6 steht.

Bei den erfindungsgemäß herzustellenden Verbindungen handelt es sich vorzugsweise um gelbe bis rote bzw. violette Farbstoffe, die zum Färben von synthetischen Fasermaterialien aus beispielsweise Polyester- oder Polyvinylmaterialien, insbesondere aber zum Massefärben von Kunststoffen eingesetzt werden. Diese Farbstoffe sind besonders hitzestabil und lichtecht.

Bei der Kondensation der Verbindungen (II) und (III) ist es nicht erforderlich eine der beiden Verbindungen im Überschuss einzusetzen, vorzugsweise wird stöchiometrisch gearbeitet.

Die Ausgangsverbindungen der Formel (II) und (III) bzw. die funktionellen Derivate von (II) in Form der Dicarbonsäureanhydride oder C₁-C₄-Alkylester, wie beispielsweise Methyl- oder Ethylester, sind bekannt oder nach bekannten Methoden erhältlich. Als aromatische Dicarbonsäuren der Formel (II) sind bevorzugt Phthalsäure, 3- oder 4-Chlorphthalsäure, Dichlorphthalsäuren, Trichlorphthalsäuren, Tetrachlorphthalsäure, Tetrabromphthalsäure, 3-Methylphthalsäure, 3,5-Dimethylphthalsäure, 4-Methylphthalsäure, 4-Phenoxyphthalsäure, 3-Hydroxyphthalsäure, 4-Phenylphthalsäure, 4-Phenylsulfonylphthalsäure, 3-Benzoylphthalsäure, 4-Nitrophthalsäure, 4-Acetaminophthalsäure, 3-Benzoylaminophthalsäure, 4-Aminosulfonylphthalsäure, 4-Phenoxysulfonylphthalsäure, 3-Acetoxyphthalsäure, Trimellithsäure, Naphthalin-2,3-dicarbonsäure, peri-Naphthalin-1,8-dicarbonsäure, 4-Chlor-naphthalin-1,8-dicarbonsäure, 4-Phenylmercapto-naphthalin-1,8-dicarbonsäure, 4,5-Ethylen-naphthalin-1,8-dicarbonsäure oder Anthracen-1,2-dicarbonsäure oder deren Anhydride oder Ester.

Als aromatische Diamine der Formel (III) sind bevorzugt: o-Phenylendiamin, Chlor-o-phenylendiamine, Dichlor-o-phenylendiamine, Methyl-o-phenylendiamine, Ethyl-o-phenylendiamine, Methoxy-o-phenylendiamine, Acetamino-o-phenylendiamine, Phenyl-o-phenylendiamine, Naphthylen-o-diamine, ferner 1,8-Naphthylendiamin, Chlor-1,8-naphthylendiamine, Dichlor-1,8-naphthylendiamine, Methyl-1,8-naphthylendiamine, Dimethyl-1,8-naphthylendiamine, Methoxy-1,8-naphthylendiamine, Ethoxy-1,8-naphthylendiamine, Acetamino-1,8-naphthylendiamine und 1,8-Diaminoacenaphthylen.

In einer besonders bevorzugten Verfahrensvariante des erfindungsgemäßen Verfahrens wird ein gegebenenfalls substituiertes peri-Naphthylendiamin als Verbindung der Formel (III), insbesondere ein unsubstituiertes 1,8-Naphthylendiamin eingesetzt.

Sofern es sich bei der aromatischen Carbonsäure II um eine Dicarbonsäure oder ihr Derivat handelt, beträgt das Verhältnis von II zu III vorzugsweise 1 bis 1,5, insbesondere 1 bis 1,1.

Das erfindungsgemäße Verfahren wird im allgemeinen bei Temperaturen von 50 bis 160°C, vorzugsweise bei 90 bis 140°C durchgeführt. Die Umsetzung erfolgt im allgemeinen bei Normaldruck, sie kann aber auch bei vermindertem oder erhöhtem Druck, insbesondere bei 1 bis 20 bar durchgeführt werden. Wird unter erhöhtem Druck gearbeitet, so ist die Umsetzung bei Temperaturen von 105 bis 160°C bevorzugt.

In einer bevorzugten Ausführungsform enthält das Reaktionsmedium zusätzlich eine anorganische oder organische Säure.

Als anorganische Säuren kommen beispielsweise HCl, H₂SO₄, H₃PO₄ oder H₂CO₃ und als organische Säuren beispielsweise: Methansulfonsäure, Essigsäure, Ameisensäure, Propionsäure, Milchsäure oder Glykolsäure in Frage. Bevorzugt wird Salzsäure oder Schwefelsäure eingesetzt.

Im allgemeinen beträgt die Menge an Säure 0,1 bis 20 Gew.-%, bezogen auf das Reaktionsmedium.

Das Reaktionsmedium enthält vorzugsweise 50 bis 85 Gew.-% Wasser, 0,5 bis 20 Gew.-% der hydrotropen Verbindung und 0 bis 20 Gew.-% gegebenenfalls weitere Zusätze.

Unter einer hydrotropen Verbindung werden wasserlösliche Verbindungen verstanden, die die Löslichkeit einer Verbindung in Wasser erhöhen. Bevorzugte hydrotrope Verbindungen besitzen eine Wasserlöslichkeit von mehr als 200 g/l bei 30°C und sind in der Lage, die Verbindungen der Formel II und III bei der Reaktionstemperatur in Wasser zu lösen.

Als bevorzugte hydrotrope Verbindungen kommen Lactame, Lactone oder Mischungen davon in Frage.

Als Lactame sind beispielsweise γ-Butyrolactam, γ-Valerolactam, ε-Caprolactam sowie ihre N-substituierten Derivate, wie N-Methyl-γ-butyrolactam zu nennen. Als Lactone sind beispielsweise γ-Butyrolacton, γ-Valerolacton und δ-Valerolacton geeignet.

Bevorzugte hydrotrope Verbindungen sind N-Methyl-γ-butyrolactam, ε-Caprolactam und γ-Butyrolacton.

Die hydrotrope Verbindung wird im allgemeinen in einer Menge eingesetzt, die ausreicht, um die eingesetzten Diamine III und/oder die Dicarbonsäuren bzw. deren Derivate II bei der gewählten Reaktionstemperatur zu lösen. Außerdem bewirken die hydrotropen Verbindungen bei hochkonzentrierten Ansätzen eine deutliche Verbesserung der Rührfähigkeit. Bevorzugt ist die hydrotrope Verbindung in einer Menge von 0,5 bis 20 Gew.-%, bezogen auf das Reaktionsmedium, insbesondere 1 bis 20 Gew.-% einzusetzen.

Das erfindungsgemäße Verfahren wird in der Regel so durchgeführt, dass man eine Lösung der Ausgangsprodukte mit Hilfe der hydrotropen Verbindung herstellt, sie gegebenenfalls mit einer anorganischen oder organischen Säure versetzt und auf eine Temperatur von 50 bis 160°C erhitzt.

Die jeweiligen Ausgangsprodukte können auch zunächst einzeln in Lösung gebracht werden, wobei vorzugsweise wenigstens eine der Lösungen wässrig ist, und dann die Lösungen vereinigt werden

Nach beendeter Reaktion wird das Produkt abfiltriert und vorzugsweise mit Wasser oder einem wässrigen Gemisch, das in der Zusammensetzung dem Reaktionsmedium entspricht, gewaschen und getrocknet.

Die so erhaltenen Verbindungen der Formel (I) entstehen in der Regel mit einer Ausbeute von 90 bis 98 %. Sie weisen einen Gehalt an nicht umgesetztem Diamin III von kleiner 500 ppm im Feststoff auf.

Die Reaktion wird vorzugsweise in einer Inertgasatmosphäre durchgeführt.

Die nach dem erfindungsgemäßen Verfahren hergestellten Farbstoffe werden zum Färben von synthetischen, hydrophoben Fasermaterialien, wie Zellulosetriacetat, Polyethylenterephthalat oder Polyamiden nach bekannten Färbeverfahren eingesetzt, insbesondere aber zum Spinnund Massefärben von thermoplastischen Kunststoffen wie Polystyrol, Polyester, Polycarbonat sowie von Mischpolymerisaten wie Acrylnitril-Butadien-Styrol, Styrolacrylnitril usw.

Unter Massefärben werden hierbei insbesondere Verfahren verstanden, bei denen der Farbstoff in die geschmolzene Kunststoffmasse eingearbeitet wird, z.B. unter Zuhilfenahme eines Extruders, oder bei denen der Farbstoff bereits Ausgangskomponenten zur Herstellung des Kunststoffs, z.B. Monomeren vor der Polymerisation, zugesetzt wird.

Besonders bevorzugte Kunststoffe sind Thermoplaste, beispielsweise Vinylpolymere, Polyester und Polyamide.

Geeignete Vinylpolymere sind Polystyrol, Styrol-Acrylnitril-Copolymere, Styrol-Butadien-Copolymere, Styrol-Butadien-Acrylnitril-Terpolymere, Polymethacrylat u.a..

Weiterhin geeignete Polyester sind: Polyethylenterephthalate, Polycarbonate und Celluloseether.

Bevorzugt sind Polystyrol, Styrol-Mischpolymere, Polycarbonate und Polymethacrylat. Besonders bevorzugt ist Polystyrol.

Die erwähnten hochmolekularen Verbindungen können einzeln oder in Gemischen, als plastische Massen oder Schmelzen vorliegen.

Die Farbstoffe werden in feinverteilter Form zur Anwendung gebracht, wobei Dispergiermittel mitverwendet werden können aber nicht müssen.

Werden die Farbstoffe (I) nach der Polymerisation eingesetzt, so werden sie mit dem Kunststoffgranulat trocken vermischt oder vermahlen und dieses Gemisch z.B. auf Mischwalzen oder in Schnecken plastifiziert und homogenisiert. Man kann die Farbstoffe aber auch der schmelzflüssigen Masse zugeben und diese durch Rühren homogen verteilen. Das derart vorgefärbte Material wird dann wie üblich z.B. durch Verspinnen zu Borsten, Fäden usw. oder durch Extrusion oder im Spritzguss-Verfahren zu Formteilen weiterverarbeitet.

Die Farbstoffe der Formel (I) werden vorzugsweise zum Färben der genannten Polymeren in Mengen von 0,0001 bis 1 Gew.-%, insbesondere 0,01 bis 0,5 Gew.-%, bezogen auf die Polymermenge, eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

Nach dem Verfahren erhält man transparente bzw. gedeckte Färbungen mit guter Hitzebeständigkeit sowie guter Licht- und Wetterechtheit.

### Beispiele

### Beispiel 1

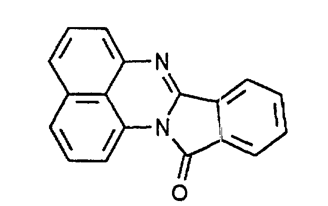

Eine Mischung aus 31,6 g (0,2 mol) 1,8-Diaminonaphthalin, 35 g ε-Caprolactam und 29,6 g (0,2 mol) Phthalsäureanhydrid wurde mit 350 ml 5%iger wäßriger Schwefelsäure versetzt und auf 80°C erwärmt. Nach 1 Stunde wurde die Temperatur auf 90 bis 95°C erhöht und 7 Stunden gehalten. Nach Beendigung der Reaktion wurde der orange Niederschlag heiß abfiltriert und mit Wasser und Methanol gewaschen. Es wurden 53 g (98 % der Theorie) des obigen Farbstoffs erhalten. Der Amingehalt wurde mittels HPLC bestimmt (< 500 ppm). 1 g des Farbstoffs wurden mit 100 ml Xylol versetzt und für 30 Min. auf 115°C erhitzt. Nach Filtration über einen Membranfilter wurde ein Filterrückstand von deutlich unter 1 % gefunden.

### Beispiel 2

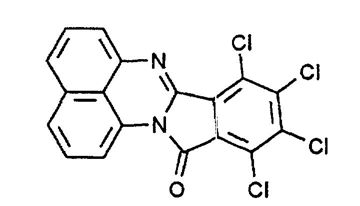

Eine Lösung aus 39 g N-Methyl-γ-butyrolactam und 25,3 g (0,16 mol) 1,8-Diaminonaphthalin wurde unter Inertgasatmosphäre mit 390 ml 10 %iger wäßriger Schwefelsäure versetzt und 30 Minuten gerührt. Anschließend wurden 45,7 g (0,16 mol) Tetrachlorphthalsäureanhydrid zudosiert und das Reaktionsgemisch in einem Autoklaven 8 Stunden auf 130°C erhitzt (Druck: 4 bar). Nach beenderter Umsetzung wurde der entstandene rote Farbstoff heiß abfiltriert und mit Wasser gewaschen. Man erhielt 63,3 g (97 % der Theorie) des obigen Farbstoffs. Der nach HPLC bestimmte Amingehalt lag unterhalb von 500 ppm. 1 g des Farbstoffes wurde mit 200 ml Xylol versetzt und für 30 Min. auf 115°C erhitzt. Nach Filtration über einen Membranfilter wurde ein Filterrückstand von deutlich <1% gefunden.

### Beispiel 3

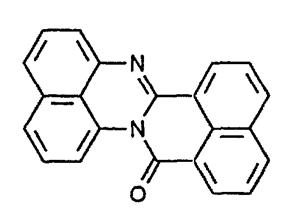

Eine Mischung aus 15,8 g (0,1 mol) 1,8-Diaminonaphthalin, 25 g N-Methyl-γ-Butyrolactam und 250 ml Wasser wurden unter Stickstoffatmosphäre mit einer Lösung aus 19,8 g (0,1 mol) Naphthalsäureanhydrid in 25 g konzentrierter Schwefelsäure versetzt und in einem Autoklaven 8 Stunden auf 150°C erhitzt. Der entstandene Farbstoff wurde abfiltriert und mit Wasser gewaschen. Man erhielt 31,4 g (98 % der Theorie) des obigen Farbstoffs. Der Amingehalt wurde mittels HPLC zu < 500 ppm ermittelt. 1 g des Farbstoffs wurden mit 100 ml Xylol versetzt und für 30 Min. auf 115°C erhitzt. Nach Filtration über einen Membranfilter wurde ein Filterrückstand von deutlich unter 1 % gefunden.

### Beispiel 4

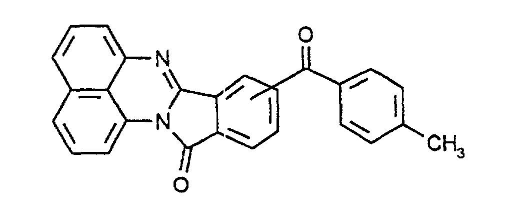

Eine Mischung aus 250 ml 5 %iger wäßriger Schwefelsäure und 28,4 g (0,1 mol) 4-(p-Toluol)phthalsäure wurde unter Stickstoffatmosphäre mit einer Lösung von 15,8 g (0,1 mol) 1,8-Diaminonaphthalin in 25 g N-Methyl-γ-butyrolactam versetzt, in einem Autoklaven auf 140°C erhitzt und die Temperatur 7 Stunden gehalten. Nach Beendigung der Reaktion wurde das Produkt heiß abgesaugt, mit einer 10 %igen wäßrigen N-Methyl-γ-butyrolactam-Lösung und anschließend mit Wasser gewaschen. Man erhielt 35,5 g (90 % d.Th.) des obigen Farbstofffs. Der Amingehalt wurde mittels HPLC bestimmt zu < 500 ppm. 1 g des Farbstoffs wurden mit 100 ml Xylol versetzt und für 30 Min. auf 115°C erhitzt. Nach Filtration über einen Membranfilter wurde ein Filterrückstand von deutlich unter 1 % gefunden.

### Beispiel 5

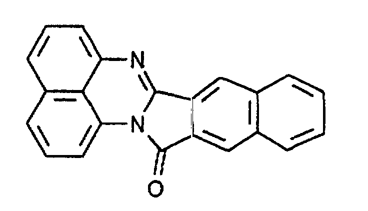

Eine Mischung aus 300 ml 6%iger wäßrige Salzsäure, 15,8 g (0,1 mol) 1,8-Diaminonaphthalin, 21,6 g (0,1 mol) 2,3-Naphthalindicarbonsäure und 40 g γ-Butyrolacton wurde 7 Stunden auf 140°C erhitzt. Nach Ende der Reaktion wurde der Farbstoff abfiltriert und mit Wasser gewaschen. Es wurden 31,1 g des obigen Farbstoffs (Δ 97 % der Theorie) erhalten. Der nach HPLC bestimmte Amingehalt wurde zu <500 ppm ermittelt. Der wie in Beispiel 1 bestimmte Filterrückstand betrug deutlich < 1 %.

### Beispiel 6

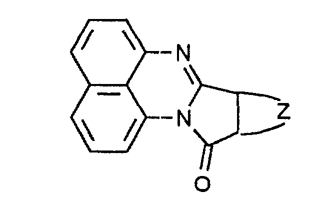

worin Z = Rest zur Vervollständigung eines 1,2-Naphthylenringes.

Eine Lösung 15,8 g (0,1 mol) 1,8-Diaminonaphthalin und 21,6 g (0,1 mol) 1,2-Naphthalindicarbonsäure in 40 g γ-Valerolactam wurde unter Stickstoffatmosphäre mit 300 ml einer 5 %igen wäßrigen Methansulfonsäure-Lösung versetzt, im Autoklaven auf 145°C erhitzt und 8 Stunden bei dieser Temperatur gerührt. Der entstandene Farbstoff wurde heiß abgesaugt und mit Wasser gewaschen. Es wurden 31 g (97% d.Th.) des obigen Farbstoffes erhalten. Der mittels HPLC bestimmte Amingehalt lag unter 500 ppm. Der wie in Bsp. 1 bestimmte Filterrückstand betrug < 1 %.

### Beispiele 7-14

In ähnlich guten Ausbeuten wurden Farbstoffe durch Umsetzung der folgenden Diamine und Dicarbonsäurederivate, analog den vorausgegangenen Beispielen, erhalten:

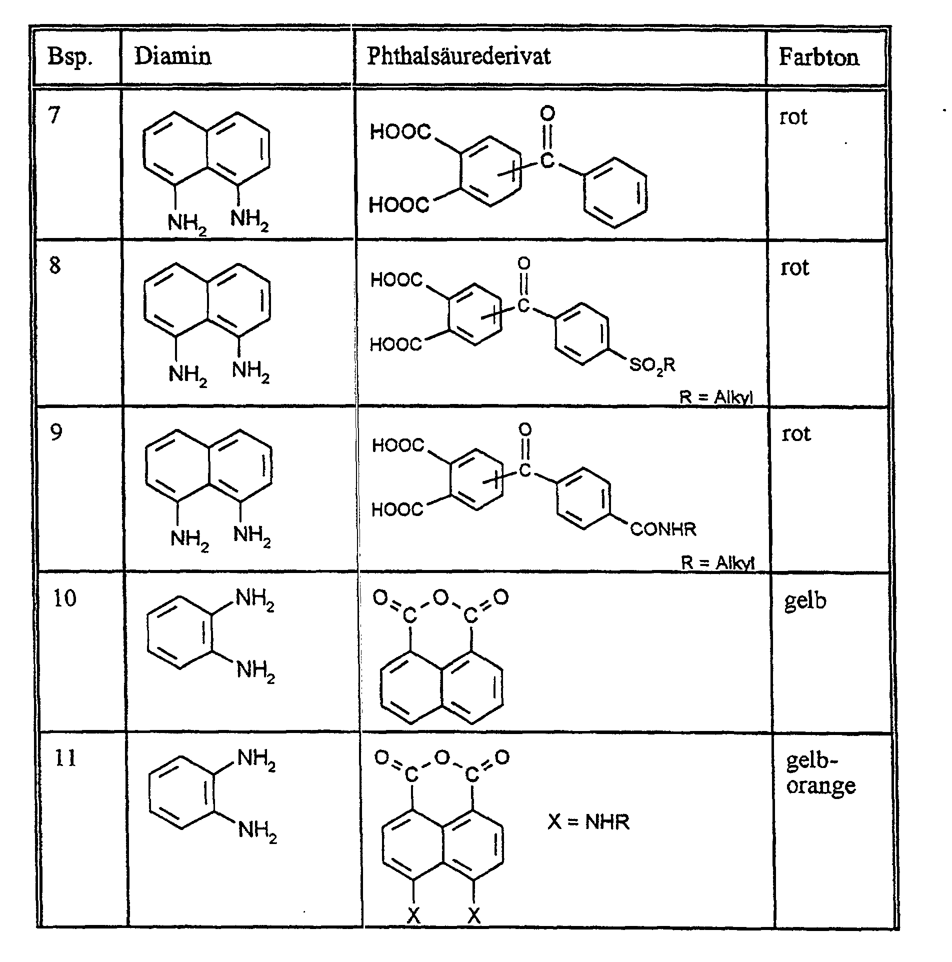

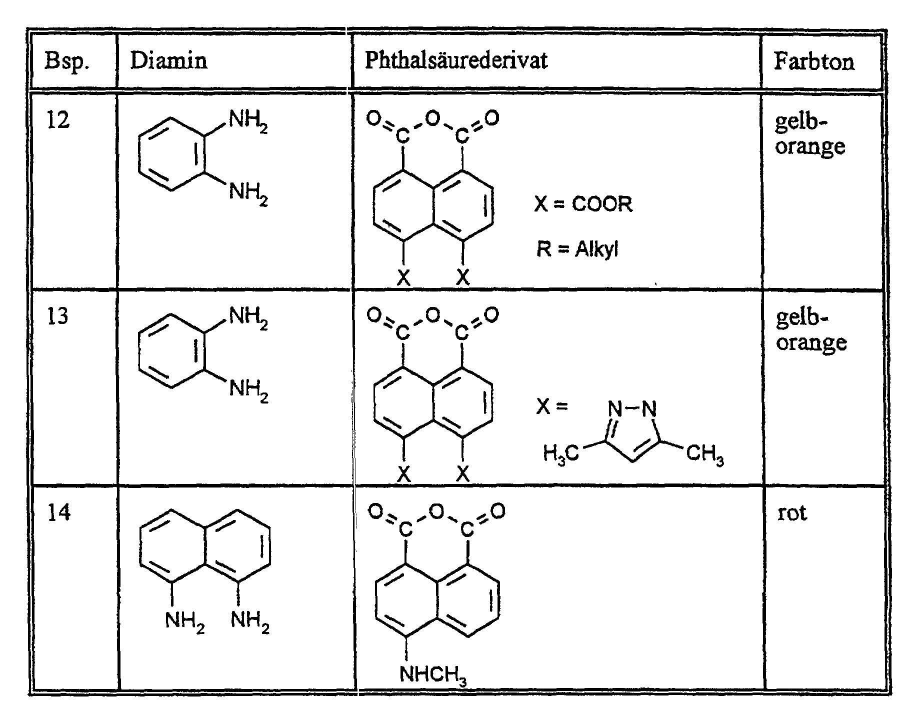

### Vergleichsbeispiel 1

Aus der Patentschrift JA 5 285 218 wurde das Beispiel 2 nachgearbeitet.

15,8 g (0,1 mol) 1,8-Diaminonaphthalin, 28,6 g (0,1 mol) Tetrachlorphthalsäureanhydrid, 20 g Ethylenglykol und 400 ml 10 %ige wäßrige Salzsäure wurden in einem Glaskolben 8 h zum Rückfluß erhitzt. Nach Filtration wurde der Filterkuchen gründlich mit Wasser gewaschen. Der erhaltene Farbstoff (38 g) besaß eine Reinheit von nur 85 % (HPLC). 1 g des Rohfarbstoffes wurde in 200 ml Xylol für 30 Minuten auf 115°C erhitzt. Nach Filtration über einen Membranfilter wurden 11 % Rückstand bezogen auf die Einwage gefunden.

### Vergleichsbeispiel 2

Aus der Patentschrift JA 5 321 221 wurde das Beispiel 1 nachgearbeitet.

15,8 g (0,1 mol) 1,8-Diaminonaphthalin, 14,8 g (0,1 mol) Phthalsäureanhydrid, 350 g 1,2 %ige wäßrige Salzsäure und 0,2 g eines nichtionischen Tensids wurden in einem Glaskolben 8 h zum Rückfluß erhitzt. Nach Filtration wurde der Filterkuchen gründlich mit Wasser gewaschen. Der erhaltene Farbstoff (25 g) besaß eine Reinheit von nur 80 % (HPLC). 1 g des Rohfarbstoffes wurden in 100 ml Xylol 30 Minuten auf 115°C erhitzt. Nach Filtration über einen Membranfilter wurden 15 % Rückstand bezogen auf die Einwage gefunden.

## Patentansprüche

1. Verfahren zur Herstellung von Verbindungen der allgemeinen Formel (I) 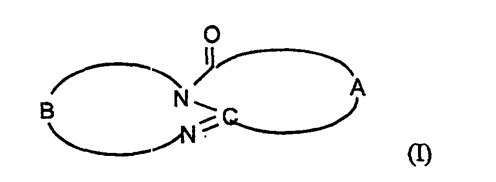 durch Umsetzung von aromatischen Dicarbonsäuren der Formel (II) 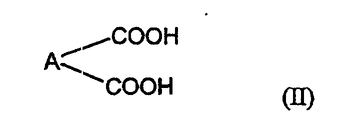 bzw. deren Anhydriden und/oder Ester mit aromatischen Diaminen der Formel (III) 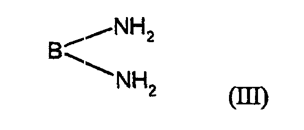 worin
A= ortho-Phenylen, ortho-Naphthylen, peri-(1,8)-Naphthylen oder Arylen aus mehr als zwei miteinander kondensierten Benzolringen, das gegebenenfalls substituiert ist, und
B= ortho-Phenylen, ortho-Naphthylen, peri-(1,8)-Naphthylen oder Arylen aus mehr als zwei miteinander kondensierten Benzolringen, das gegebenenfalls substituiert ist, bedeuten
wobei die Arylenreste A und B in Formel (I), die mehr als zwei miteinander kondensierte Benzolringe aufweisen, in ortho- oder entsprechend einer peri-Stellung im Naphthalin verbrückt sind, **dadurch gekennzeichnet**, dass die Umsetzung von (II) mit (III) in einem Reaktionsmedium erfolgt, welches Wasser und eine Hydrotrope Verbindung, die eine relative Dielektrizitätskonstante ε von 20 bis 60 (bei 25°C) besitzt, enthält.

2. Verfahren gemäss Anspruch 1, **dadurch gekennzeichnet**, dass das Reaktionsmedium zusätzlich eine anorganische oder organische Säure enthält.

3. Verfahren gemäss Anspruch 1, **dadurch gekennzeichnet**, dass das Verfahren bei einer Temperatur von 50 bis 160°C durchgeführt wird.

4. Verfahren gemäss Anspruch 1, **dadurch gekennzeichnet**, dass als hydrotrope Verbindung ein Lactam, Lacton, Amid oder Mischungen davon eingesetzt werden.

5. Verfahren gemäß Anspruch 1, **dadurch gekennzeichnet**, dass die hydrotrope Verbindung in einer Menge von 0,1 bis 20 Gew.-%, bezogen auf das Reaktionsmedium eingesetzt wird.

## Claims

1. Process for the preparation of compounds of the general formula (I) 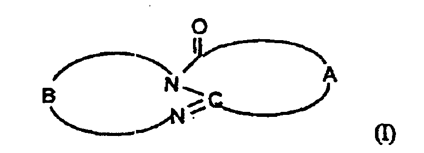 by reaction of aromatic dicarboxylic acids of the formula (II) 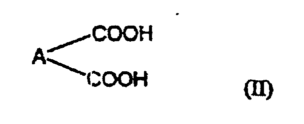 or anhydrides and/or esters thereof with aromatic diamines of the formula (III) 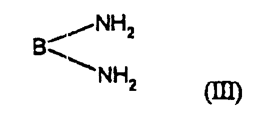 wherein
A = ortho-phenylene, ortho-naphthylene, peri-(1,8)-naphthylene or arylene of more than two benzene rings fused with one another, which is optionally substituted, and
B = ortho-phenylene, ortho-naphthylene, peri-(1,8)-naphthylene or arylene of more than two benzene rings fused with one another, which is optionally substituted,
where the arylene radicals A and B in formula (I) which contain more than two benzene rings fused with one another are bridged in the ortho-position or corresponding to a peri-position in naphthalene, **characterized in that** the reaction of (II) with (III) is carried out in a reaction medium which comprises water and a hydrotropic compound which has a relative dielectric constant ε of 20 to 60 (at 25°C).

2. Process according to Claim 1, **characterized in that** the reaction medium additionally comprises an inorganic or organic acid.

3. Process according to Claim 1, **characterized in that** the process is carried out at a temperature from 50 to 160°C.

4. Process according to Claim 1, **characterized in that** a lactam, lactone, amide or mixtures thereof are employed as the hydrotropic compound.

5. Process according to Claim 1, **characterized in that** the hydrotropic compound is employed in an amount of 0.1 to 20% by weight, based on the reaction medium.

## Revendications

1. Procédé pour la préparation des composés de formule générale (I) 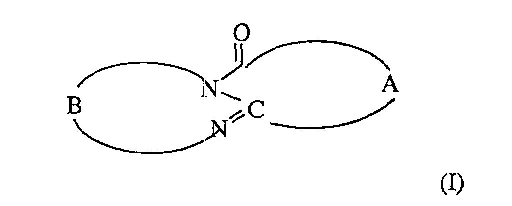 par réaction d'acides dicarboxyliques aromatiques de formule (II) 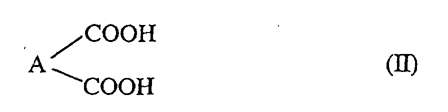 ou de leurs anhydrides et/ou esters, avec des diamines aromatiques de formule (III) 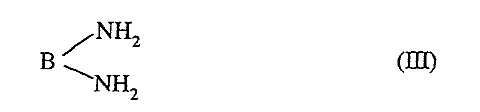 les symboles A et B ayant les significations suivantes:
A = ortho-phénylène, ortho-naphtylène, péri-(1,8)-naphtylène ou arylène comportant plus de deux cycles benzéniques condensés entre eux, chacun de ces groupes étant éventuellement substitué, et
B = ortho-phénylène, ortho-naphtylène, péri-(1,8)-naphtylène ou arylène comportant plus de deux cycles benzéniques condensés entre eux, chacun de ces groupes étant éventuellement substitué,
les groupes arylène A et B de la formule (I) comportant plus de deux cycles benzéniques condensés entre eux, étant eux-mêmes reliés par un pont en position ortho ou, en correspondance, en position péri du naphtalène, ce procédé se caractérisant en ce que l'on procède à la réaction entre (II) et (III) dans un milieu de réaction qui contient de l'eau et un composé hydrotrope ayant une constante diélectrique relative ε de 20 à 60 (à 25°C).

2. Procédé selon la revendication 1, **caractérisé en ce que** le milieu de réaction contient en outre un acide minéral ou organique.

3. Procédé selon la revendication 1, **caractérisé en ce que** l'on opère à des températures de 50 à 160°C.

4. Procédé selon la revendication 1, **caractérisé en ce que** le composé hydrotrope utilisé est un lactame, une lactone, un amide ou un mélange de tels composés.

5. Procédé selon la revendication 1, **caractérisé en ce que** le composé hydrotrope est mis en oeuvre en quantité de 0,1 à 20 % du poids du milieu de réaction.
